# EUROPÄISCHE PATENTANMELDUNG

(11) **EP 1 167 520 A2**
(43) Veröffentlichungstag der Anmeldung: **02.01.2002**
(21) Anmeldenummer: 01114629.7
(22) Anmeldetag: 19.06.2001
(51) Int. Cl.: C12N 9/12, C12N 9/10, C12N 9/00, C12N 9/88, C12N 15/54, C12N 15/60, C12N 15/52, C12P 7/42

(54) **Verfahren zur fermentativen Herstellung von D-Pantothensäure unter Verwendung coryneformer Bakterien**

(30) Priorität: 23.06.2000 DE 10030702
(71) Anmelder: Degussa AG, 40474 Düsseldorf (DE)
(72) Erfinder: Dusch, Nicole, Dr., 33824 Werther (DE); Thierbach, Georg, Dr., 33613 Bielefeld (DE)

(57) **Zusammenfassung**

Die Erfindung betrifft ein Verfahren zur Herstellung von D-Pantothensäure durch Fermentation coryneformer Bakterien, bei dem man Bakterien einsetzt, in denen man die für die Phosphofructokinase (EC 2.7.1.11) kodierende Nucleotidsequenz (pfkA-Gen) verstärkt, insbesondere überexprimiert, wobei man folgende Schritte ausführt:
a) Fermentation der D-Pantothensäure produzierenden Bakterien, in denen zumindest das für Phosphofructokinase kodierende Gen verstärkt wird,
b) Anreicherung der D-Pantothensäure im Medium oder in den Zellen der Bakterien und
c) Isolieren der produzierten D-Pantothensäure.

SEQUENZPROTOKOLL

<110> Degussa-Hüls AG

<120> Verfahren zur fermentativen Herstellung von D-Pantothensäure
      unter Verwendung coryneformer Bakterien

<130> 000213 BT

<140>
<141>

<160> 2

<170> PatentIn Ver. 2.1

<210> 1
<211> 1274
<212> DNA
<213> Corynebacterium glutamicum

<220>
<221> CDS
<222> (143)..(1171)

<400> 1
gtcgatttgt taatgaaact gcagctctgg cgattaaata agatggtcag agacagtttt 60

ttggcctgtc aacccctgtg attctcttat ttttgggtga ttgttccggc gcgggtgttg 120

tgatgggttt aatatggaag ac atg cga att gct act ctc acg tca ggc ggc 172
                         Met Arg Ile Ala Thr Leu Thr Ser Gly Gly
                           1               5                  10

gac tgc ccc gga cta aac gcc gtc atc cga gga atc gtc cgc aca gcc 220
Asp Cys Pro Gly Leu Asn Ala Val Ile Arg Gly Ile Val Arg Thr Ala
                 15                  20                  25

agc aat gaa ttt ggc tcc acc gtc gtt ggt tat caa gac ggt tgg gaa 268
Ser Asn Glu Phe Gly Ser Thr Val Val Gly Tyr Gln Asp Gly Trp Glu
             30                  35                  40

gga ctg tta ggc gat cgt cgc gta cag ctg tat gac gat gaa gat att 316
Gly Leu Leu Gly Asp Arg Arg Val Gln Leu Tyr Asp Asp Glu Asp Ile
         45                  50                  55

gac cga atc ctc ctt cga ggc ggc acc att ttg ggc act ggt cgc ctc 364
Asp Arg Ile Leu Leu Arg Gly Gly Thr Ile Leu Gly Thr Gly Arg Leu
     60                  65                  70

cat ccg gac aag ttt aag gcc gga att gat cag att aag gcc aac tta 412
His Pro Asp Lys Phe Lys Ala Gly Ile Asp Gln Ile Lys Ala Asn Leu
 75                  80                  85                  90

gaa gac gcc ggc atc gat gcc ctt atc cca atc ggt ggc gaa gga acc 460
Glu Asp Ala Gly Ile Asp Ala Leu Ile Pro Ile Gly Gly Glu Gly Thr
                 95                 100                 105

ctg aag ggt gcc aag tgg ctg tct gat aac ggt atc cct gtt gtc ggt 508
Leu Lys Gly Ala Lys Trp Leu Ser Asp Asn Gly Ile Pro Val Val Gly
            110                 115                 120

gtc cca aag acc att gac aat gac gtg aat ggc act gac ttc acc ttc 556
Val Pro Lys Thr Ile Asp Asn Asp Val Asn Gly Thr Asp Phe Thr Phe
        125                 130                 135

ggt ttc gat act gct gtg gca gtg gct acc gac gct gtt gac cgc ctg 604
Gly Phe Asp Thr Ala Val Ala Val Ala Thr Asp Ala Val Asp Arg Leu
    140                 145                 150

cac acc acc gct gaa tct cac aac cgt gtg atg atc gtg gag gtc atg 652
His Thr Thr Ala Glu Ser His Asn Arg Val Met Ile Val Glu Val Met
155                 160                 165                 170

ggc cgc cac gtg ggt tgg att gct ctg cac gca ggt atg gcc ggc ggt 700
Gly Arg His Val Gly Trp Ile Ala Leu His Ala Gly Met Ala Gly Gly
                175                 180                 185

gct cac tac acc gtt att cca gaa gta cct ttc gat att gca gag atc 748
Ala His Tyr Thr Val Ile Pro Glu Val Pro Phe Asp Ile Ala Glu Ile
            190                 195                 200

tgc aag gcg atg gaa cgt cgc ttc cag atg ggc gag aag tac ggc att 796
Cys Lys Ala Met Glu Arg Arg Phe Gln Met Gly Glu Lys Tyr Gly Ile
        205                 210                 215

atc gtc gtt gcg gaa ggt gcg ttg cca cgc gaa ggc acc atg gag ctt 844
Ile Val Val Ala Glu Gly Ala Leu Pro Arg Glu Gly Thr Met Glu Leu
    220                 225                 230

cgt gaa ggc cac att gac cag ttc ggt cac aag acc ttc acg gga att 892
Arg Glu Gly His Ile Asp Gln Phe Gly His Lys Thr Phe Thr Gly Ile
235                 240                 245                 250

gga cag cag atc gct gat gag atc cac gtg cgc ctc ggc cac gat gtt 940
Gly Gln Gln Ile Ala Asp Glu Ile His Val Arg Leu Gly His Asp Val
                255                 260                 265

cgt acg acc gtt ctt ggc cac att caa cgt ggt gga acc cca act gct 988
Arg Thr Thr Val Leu Gly His Ile Gln Arg Gly Gly Thr Pro Thr Ala
            270                 275                 280

ttc gac cgt gtt ctg gcc act cgt tat ggt gtt cgt gca gct cgt gcg 1036
Phe Asp Arg Val Leu Ala Thr Arg Tyr Gly Val Arg Ala Ala Arg Ala
        285                 290                 295

tgc cat gag gga agc ttt gac aag gtt gtt gct ttg aag ggt gag agc 1084
Cys His Glu Gly Ser Phe Asp Lys Val Val Ala Leu Lys Gly Glu Ser
    300                 305                 310

att gag atg atc acc ttt gaa gaa gca gtc gga acc ttg aag gaa gtt 1132
Ile Glu Met Ile Thr Phe Glu Glu Ala Val Gly Thr Leu Lys Glu Val
315                 320                 325                 330

cca ttc gaa cgc tgg gtt act gcc cag gca atg ttt gga tagtttttcg 1181
Pro Phe Glu Arg Trp Val Thr Ala Gln Ala Met Phe Gly
                335                 340

ggcttttatc aacagccaat aacagctctt tcgcccattg aggtggaggg gctgtttttt 1241

catgccgtaa ggaaagtgca agtaagtgaa atc 1274

<210> 2
<211> 343
<212> PRT
<213> Corynebacterium glutamicum

<400> 2
Met Arg Ile Ala Thr Leu Thr Ser Gly Gly Asp Cys Pro Gly Leu Asn
1               5                   10                  15

Ala Val Ile Arg Gly Ile Val Arg Thr Ala Ser Asn Glu Phe Gly Ser
            20                  25                  30

Thr Val Val Gly Tyr Gln Asp Gly Trp Glu Gly Leu Leu Gly Asp Arg
        35                  40                  45

Arg Val Gln Leu Tyr Asp Asp Glu Asp Ile Asp Arg Ile Leu Leu Arg
    50                  55                  60

Gly Gly Thr Ile Leu Gly Thr Gly Arg Leu His Pro Asp Lys Phe Lys
65                  70                  75                  80

Ala Gly Ile Asp Gln Ile Lys Ala Asn Leu Glu Asp Ala Gly Ile Asp
                85                  90                  95

Ala Leu Ile Pro Ile Gly Gly Glu Gly Thr Leu Lys Gly Ala Lys Trp
            100                 105                 110

Leu Ser Asp Asn Gly Ile Pro Val Val Gly Val Pro Lys Thr Ile Asp
        115                 120                 125

Asn Asp Val Asn Gly Thr Asp Phe Thr Phe Gly Phe Asp Thr Ala Val
    130                 135                 140

Ala Val Ala Thr Asp Ala Val Asp Arg Leu His Thr Thr Ala Glu Ser
145                 150                 155                 160

His Asn Arg Val Met Ile Val Glu Val Met Gly Arg His Val Gly Trp
                165                 170                 175

Ile Ala Leu His Ala Gly Met Ala Gly Gly Ala His Tyr Thr Val Ile
            180                 185                 190

Pro Glu Val Pro Phe Asp Ile Ala Glu Ile Cys Lys Ala Met Glu Arg
        195                 200                 205

Arg Phe Gln Met Gly Glu Lys Tyr Gly Ile Ile Val Val Ala Glu Gly
    210                 215                 220

Ala Leu Pro Arg Glu Gly Thr Met Glu Leu Arg Glu Gly His Ile Asp
225                 230                 235                 240

Gln Phe Gly His Lys Thr Phe Thr Gly Ile Gly Gln Gln Ile Ala Asp
                245                 250                 255

Glu Ile His Val Arg Leu Gly His Asp Val Arg Thr Thr Val Leu Gly
            260                 265                 270

His Ile Gln Arg Gly Gly Thr Pro Thr Ala Phe Asp Arg Val Leu Ala
        275                 280                 285

Thr Arg Tyr Gly Val Arg Ala Ala Arg Ala Cys His Glu Gly Ser Phe
    290                 295                 300

Asp Lys Val Val Ala Leu Lys Gly Glu Ser Ile Glu Met Ile Thr Phe
305                 310                 315                 320

Glu Glu Ala Val Gly Thr Leu Lys Glu Val Pro Phe Glu Arg Trp Val
                325                 330                 335

Thr Ala Gln Ala Met Phe Gly
            340

## Beschreibung

Gegenstand der Erfindung ist ein Verfahren zur fermentativen Herstellung von D-Pantothensäure unter Verwendung coryneformer Bakterien, in denen des pfkA-Gen verstärkt ist.

### Stand der Technik

Die Pantothensäure stellt ein kommerziell bedeutendes Vitamin dar, das in der Kosmetik, der Medizin, der Humanernährung und in der Tierernährung Anwendung findet.

Pantothensäure kann durch chemische Synthese oder biotechnisch durch Fermentation geeigneter Mikroorganismen in geeigneten Nährlösungen hergestellt werden. Bei der chemischen Synthese ist das DL-Pantolacton eine wichtige Zwischenstufe. Es wird in einem mehrstufigen Verfahren aus Formaldehyd, Isobutylaldehyd und Cyanid hergestellt. In weiteren Verfahrensschritten wird das racemische Gemisch aufgetrennt, D-Pantolacton mit β-Alanin kondensiert und so die gewünschte D-Pantothensäure erhalten.

Der Vorteil der fermentativen Herstellung durch Mikroorganismen liegt in der direkten Bildung der gewünschten stereoisomeren D-Form, die frei von L-Pantothensäure ist.

Verschiedene Arten von Bakterien, wie zum Beispiel Escherichia coli, Arthrobacter ureafaciens, Corynebacterium erythrogenes, Brevibacterium ammoniagenes und auch Hefen, wie zum Beispiel Debaromyces castellii können wie in EP-A 0 493 060 gezeigt, in einer Nährlösung, die Glucose, DL-Pantoinsäure und β-Alanin enthält, D-Pantothensäure produzieren. EP-A 0 493 060 zeigt weiterhin, dass bei Escherichia coli durch Amplifikation von Pantothensäure-Biosynthesegenen aus E.coli, die auf den Plasmiden pFV3 und pFV5 enthalten sind, in einer Nährlösung, die Glucose, DL-Pantoinsäure und β-Alanin enthält, die Bildung von D-Pantothensäure verbessert wird.

EP-A 0 590 857 und US-Patent 5,518,906 beschreiben von Escherichia coli Stamm IFO3547 abgeleitete Mutanten, wie FV5714, FV525, FV814, FV521, FV221, FV6051 und FV5069, die Resistenzen gegen verschiedene Antimetabolite wie Salizylsäure, α-Ketobuttersäure, β-Hydroxyasparaginsäure, O-Methylthreonin und α-Ketoisovaleriansäure tragen. Sie produzieren in einer Nährlösung, die Glucose enthält, Pantoinsäure, und in einer Glucose- und β-Alanin-haltigen Nährlösung D-Pantothensäure. In EP-A 0 590 857 und US-Patent 5,518,906 wird weiterhin gezeigt, dass nach Amplifikation der Pantothensäure-Biosynthesegene, die auf dem Plasmid pFV31 enthalten sind, in den oben genannten Stämmen in glucose-haltigen Nährlösungen, die Produktion von D-Pantoinsäure und in einer Nährlösung, die Glucose und β-Alanin enthält, die Produktion von D-Pantothensäure verbessert wird.

Verfahren zur Herstellung von D-Pantothensäure mit Hilfe von Corynebacterium glutamicum sind in der Literatur nur ansatzweise bekannt. So berichten Sahm und Eggeling (Applied and Environmental Microbiology 65(5), 1973-1979 (1999)) über den Einfluss der Überexpression der Gene panB und panC und Dusch et al. (Applied and Environmental Microbiology 65(4), 1530-1539 (1999)) über den Einfluß des Gens panD auf die Bildung der D-Pantothensäure.

### Aufgabe der Erfindung

Die Erfinder haben sich die Aufgabe gestellt, neue Grundlagen für verbesserte Verfahren zur fermentativen Herstellung von Pantothensäure mit coryneformen Bakterien bereitzustellen.

### Beschreibung der Erfindung

Das Vitamin Pantothensäure stellt ein kommerziell bedeutendes Produkt dar, das in der Kosmetik, der Medizin, der Humanernährung und in der Tierernährung Anwendung findet. Es besteht ein allgemeines Interesse daran, verbesserte Verfahren zur Herstellung von Pantothensäure bereitzustellen.

Wenn im Folgenden D-Pantothensäure oder Pantothensäure oder Pantothenat erwähnt werden, sind damit nicht nur die freien Säuren, sondern auch die Salze der D-Pantothensäure, wie zum Beispiel das Calcium-, Natrium-, Ammonium- oder Kaliumsalz gemeint.

Gegenstand der Erfindung ist ein Verfahren zur fermentativen Herstellung von D-Pantothensäure unter Verwendung von coryneformen Bakterien, in denen die für das Enzym Phosphofructokinase (EC 2.7.1.11) kodierende Nucleotidsequenz (pfkA-Gen) verstärkt, insbesondere überexprimiert wird.

Die eingesetzten Stämme produzieren bevorzugt bereits vor der Verstärkung des pfkA-Gens D-Pantothensäure.

Bevorzugte Ausführungsformen finden sich in den Ansprüchen.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können D-Pantothensäure aus Glucose, Saccharose, Lactose, Fructose, Maltose, Melasse, Stärke, Cellulose oder aus Glycerin und Ethanol herstellen. Es handelt sich um Vertreter coryneformer Bakterien, insbesondere der Gattung Corynebacterium. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind beispielsweise die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte, D-Pantothensäure produzierende Mutanten wie beispielsweise
Corynebacterium glutamicum ATCC13032ΔilvA/pEC7panBC
Corynebacterium glutamicum ATCC13032/pND-D2

Es wurde gefunden, dass coryneforme Bakterien nach Überexpression des für die Phosphofructokinase (EC 2.7.1.11) kodierenden pfkA-Gens in verbesserter Weise Pantothensäure produzieren.

Die Nukleotidsequenz des pfkA-Gens ist in der SEQ ID No 1 und die sich daraus ergebende Aminosäuresequenz des Enzymproteins in SEQ ID No 2 dargestellt.

Das in der SEQ ID No 1 beschriebene pfkA-Gen kann erfindungsgemäß verwendet werden. Weiterhin können Allele des pfkA-Gens verwendet werden, die sich aus der Degeneriertheit des genetischen Codes oder durch funktionsneutrale Sinnmutationen (sense mutations) ergeben.

Zur Erzielung einer Verstärkung (zum Beispiel Überexpression) erhöht man zum Beispiel die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression im Verlaufe der fermentativen Pantothensäure-Bildung zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammen-setzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15-24 (1993)), in der japanischen Offenlegungsschrift JP-A-10-229891, bei Jensen und Hammer (Biotechnology and Bioengineering 58, 191-195 (1998)) und in bekannten Lehrbüchern der Genetik und Molekularbiologie.

Beispielhaft wurde das pfkA-Gen mit Hilfe von Plasmiden überexprimiert.

Als Plasmide eignen sich solche, die in coryneformen Bakterien repliziert werden. Zahlreiche bekannte Plasmidvektoren wie zum Beispiel pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie zum Beispiel solche, die auf pCG4 (US-A 4,489,160), oder pNG2 (Serwold-Davis et al., FEMS Microbiology Letters 66, 119-124 (1990)), oder pAG1 (US-A 5,158,891) beruhen, können in gleicher Weise verwendet werden.

Weiterhin eignen sich solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen bespielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173:4510-4516) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses enthält der resultierende Stamm mindestens zwei Kopien des betreffenden Gens.

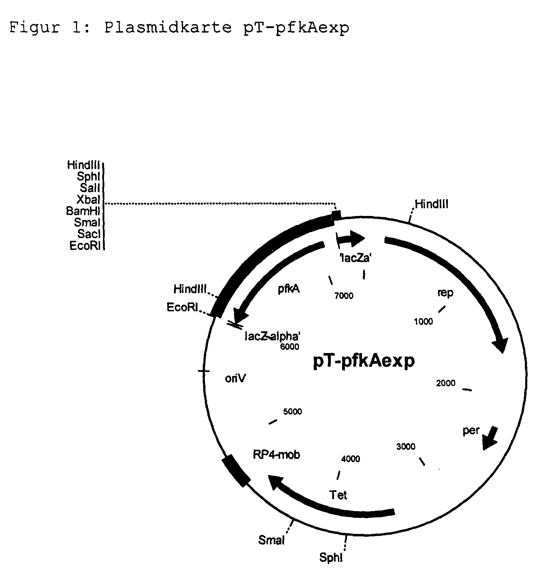

Beispielhaft wurde im Rahmen der vorliegenden Erfindung das Verfahren der Genamplifikation durch Integration in das Chromosom eingesetzt. Hierzu wurde der in der Abbildung 1 dargestellte Plasmidvektor pT-pfkAexp verwendet.

Zusätzlich kann es für die Produktion von Pantothensäure vorteilhaft sein, neben dem für die Phosphofructokinase kodierendem Gen ein oder mehrere weitere für Enzyme des Pantothensäure-Biosyntheseweges oder des Keto-Isovaleriansäure-Biosyntheseweges codierende Gene wie zum Beispiel
- das für die Ketopantoat-Hydroxymethyltransferase kodierende panB-Gen (Sahm et al., Applied and Environmental Microbiology, 65, 1973-1979 (1999)) oder
- das für die Pantothenat-Synthetase kodierende panC-Gen (Sahm et al., Applied and Environmental Microbiology, 65, 1973-1979 (1999)) oder
- das für die Dihydroxysäure-Dehydratase kodierende ilvD-Gen
zu verstärken, insbesondere zu überexprimieren.

Weiterhin kann es für die Produktion von Pantothensäure vorteilhaft sein, neben der Überexpression der Phosphofructokinase unerwünschte Nebenreaktionen auszuschalten (Nakayama: "Breeding of Amino Acid Producing Micro-organisms"', in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Pantothensäure-Produktion kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate, wie zum Beispiel Glucose, Saccharose, Lactose, Fructose, Maltose, Melasse, Stärke und Cellulose, Öle und Fette wie zum Beispiel Sojaöl, Sonnenblumenöl, Erdnussöl und Kokosfett, Fettsäuren wie zum Beispiel Palmitinsäure, Stearinsäure und Linolsäure, Alkohole wie zum Beispiel Glycerin und Ethanol und organische Säuren wie zum Beispiel Essigsäure verwendet werden. Diese Stoffe können einzeln oder als Mischung verwendet werden. Als Stickstoffquelle können organische, Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium-haltigen Salze verwendet werden. Das Kulturmedium muß weiterhin Salze von Metallen enthalten, wie zum Beispiel Magnesiumsulfat oder Eisensulfat, die für das Wachstum notwendig sind. Schließlich können essentielle Wuchsstoffe, wie Aminosäuren und Vitamine zusätzlich zu den oben genannten Stoffen eingesetzt werden. Dem Kulturmedium können überdies zur zusätzlichen Steigerung der Pantothensäure-Produktion Vorstufen der Pantothensäure, wie Aspartat, β-Alanin, Ketoisovalerat, Ketopantoinsäure oder Pantoinsäure, und gegebenenfalls deren Salze zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, zum Beispiel Antibiotika, hinzugefügt werden. Um aerobe Bedingungen aufrechtzuerhalten werden Sauerstoff oder Sauerstoffhaltige Gasmischungen, wie zum Beispiel Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum an Pantothensäure gebildet hat. Dieses Ziel wird normalerweise innerhalb von 10 Stunden bis 160 Stunden erreicht.

Die Konzentration an gebildeter Pantothensäure kann mit bekannten chemischen (Velisek; Chromatographic Science 60, 515-560 (1992)) oder mikrobiologischen Verfahren wie zum Beispiel dem Lactobacillus plantarum Test (DIFCO MANUAL, 10^{th} Edition, S. 1100-1102; Michigan, USA) bestimmt werden.

Folgender Mikroorganismus wurde bei der Deutschen Sammlung für Mikroorganismen und Zellkulturen (DSMZ, Braunschweig, Deutschland) gemäß Budapester Vertrag hinterlegt:
Corynebacterium glutamicum DSM5715/pT-pfkAexp als DSM13253

### Beispiele

Die vorliegende Erfindung wird im folgenden anhand von Ausführungsbeispielen näher erläutert.

Zu diesem Zweck wurden Versuche mit dem Isoleucinbedürftigen Stamm ATCC13032ΔilvA und dem Plasmid pND-D2 durchgeführt. Der Stamm ATCC13032ΔilvA ist als DSM12455 bei der Deutschen Sammlung für Mikroorganismen und Zellkulturen in Braunschweig (Deutschland) gemäss Budapester Vertrag hinterlegt worden. Das panD-Gen haltige Plasmid pND-D2 ist bei Dusch et al. (Applied and Environmental Microbiology 65(4), 1530-1539 (1999)) beschrieben und in Form des Stammes Corynebacterium glutamicum ATCC13032/pND-D2 als DSM12438 ebenfalls bei der Deutschen Sammlung für Mikroorganismen und Zellkulturen in Braunschweig (Deutschland) gemäss Budapester Vertrag hinterlegt.

### Beispiel 1

Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCos1 Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC 13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 µg/ml Ampicillin ausplattiert wurden. Nach Inkubation über Nacht bei 37°C wurden rekombinante Einzelklone selektioniert.

### Beispiel 2

Isolierung und Sequenzierung des pfkA-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 µg/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems (Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurde mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID NO 1 dargestellt. Die Analyse der Nukleotidsequenz ergab ein offenes Leseraster von 1029 Basenpaaren, welches als pfkA-Gen bezeichnet wurde. Das pfkA-Gen kodiert für ein Protein von 343 Aminosäuren dargestellt in SEQ ID NO 2.

### Beispiel 3

Herstellung eines Plasmides zur Expression von pfkA in Corynebacterium glutamicum

### 3.1. Klonierung von pfkA im Vektor pCR-Blunt2

Chromosomale DNA aus Corynebacterium glutamicum ATCC 13032 wurde wie bei Tauch et al. (1995, Plasmid 33:168-179). Aufgrund der aus Beispiel 2 für C. glutamicum bekannten Sequenz des pfkA-Gens wurden die folgenden Oligonukleotide für die Polymerase Kettenreaktion ausgewählt:
pfkA-exp
5'-AAC TGC AGC TCT GGC GAT TA-3'
pfk-ex2
5'-AAC TAT CCA AAC ATT GCC TG-3'

Die dargestellten Primer wurden von der Firma MWG Biotech (Ebersberg, Deutschland) synthetisiert und nach der Standard-PCR-Methode von Innis et al. (PCR protocols. A guide to methods and applications, 1990, Academic Press) mit Pwo-Polymerase der Firma Roche Diagnostics GmbH (Mannheim, Deutschland) die PCR Reaktion durchgeführt. Mit Hilfe der Polymerase-Kettenreaktion wurde ein ca. 1160 bp großes DNA-Fragment isoliert, welches das pfkA-Gen trägt.

Das amplifizierte DNA Fragment wurde mit dem Zero Blunt TOPO PCR Cloning Kit der Firma Invitrogen Corporation (Carlsbad, CA, USA; Katalog Nummer K2800-20) in den Vektor pCR-Blunt II-TOPO Vektor (Shuman et al., (1994) Journal of Biological Chemistry. 269:32678-32684; Bernard et al., (1983) Journal of Molecular Biology. 234:534-541) ligiert. Anschließend wurde der E. coli Stamm Top10 (Grant et al. (1990) Proceedings of the National Academy of Sciences, USA. 87:4645-4649) mit dem Ligationsansatz transformiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y.), der mit 50 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und anschließender Agarosegel-Elektrophorese (0,8%) überprüft. Das Plasmid wurde pCRB1-pfkAexp1 genannt.

### 3.2. Herstellung des Shuttle Vektors pEC-T18mob2

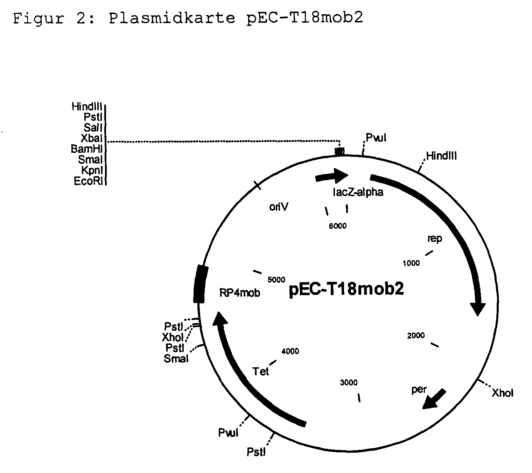

Nach dem Stand der Technik wurde der E. coli - C. glutamicum Shuttle-Vektor konstruiert. Der Vektor enthält die Replikationsregion rep des Plasmids pGA1 einschließlich des Replikationseffectors per (US-A- 5,175,108; Nesvera et al., Journal of Bacteriology 179, 1525-1532 (1997)), das Tetracyclinresistenz vermittelnde tetA(Z)-Gen des Plasmids pAG1 (US-A- 5,158,891; Genbank-Eintrag beim National Center for Biotechnology Information (NCBI, Bethesda, MD, USA) mit der accession number AF121000), die Replikationsregion oriV des Plasmids pMB1 (Sutcliffe, Cold Spring Harbor Symposium on Quantitative Biology 43, 77-90 (1979)), das lacZα Genfragment einschließlich des lac-Promotors und einer Mehrfachklonierschnittstelle (multiple cloning site, mcs) (Norrander et al. Gene 26, 101-106 (1983)) und die mob-Region des Plasmids RP4 (Simon et al.,(1983) Bio/Technology 1:784-791). Der konstruierte Vektor wurde in den E. coli Stamm DH5α (Hanahan, In: DNA cloning. A practical approach. Vol. I. IRL-Press, Oxford, Washington DC, USA) transformiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y.), der mit 5 mg/l Tetracyclin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und HindIII anschließender Agarosegel-Elektrophorese (0,8%) überprüft. Das Plasmid wurde pEC-T18mob2 genannt und ist in Figur 2 dargestellt.

### 3.3. Klonierung von pfkA im Shuttle-Vektor pEC-T18mob2

Als Vektor wurde der in Beispiel 3.2 beschriebene E. coli - C. glutamicum Shuttle-Vektor pEC-T18mob2 verwendet. DNA dieses Plasmids wurde mit dem Restriktionsenzym EcoRI vollständig gespalten und anschließend mit shrimp alkalischer Phosphatase (Roche Molecular Biochemicals, Mannheim, Deutschland, Produktbeschreibung SAP, Product No. 1758250) dephosphoryliert.

Aus dem in Beispiel 3.1. beschriebenen Plasmid pCRB1-pfkAexp1 wurde das pfkA-Gen durch vollständige Spaltung mit dem Enzym EcoRI isoliert. Das ca 1160bp große pfkA Fragment wurde aus dem Agarosegel mit dem QiaExII Gel Extraction Kit (Product No. 20021, Qiagen, Hilden, Germany) isoliert.

Das auf diese Weise gewonnene pfkA-Fragment wurde mit dem vorbereiteten Vektor pEC-T18mob2 gemischt und der Ansatz mit T4-DNA-Ligase (Amersham Pharmacia, Freiburg, Deutschland, Produktbeschreibung T4-DNA-Ligase, Code no.27-0870-04) behandelt. Der Ligationsansatz wurde in den E. coli Stamm DH5αmcr (Grant et al., (1990). Proceedings of the National Academy of Sciences USA. 87: 4645-4649) transformiert. Die Selektion von Plasmid-tragenden Zellen erfolgte durch Ausplattieren des Transformationsansatzes auf LB-Agar (Lennox, 1955, Virology, 1:190) mit 5 mg/l Tetracyclin. Nach Inkubation über Nacht bei 37°C wurden rekombinante Einzelklone selektioniert. Plasmid DNA wurde aus einer Transformante mit dem Qiaprep Spin Miniprep Kit (Product No. 27106, Qiagen, Hilden, Germany) nach Herstellerangaben isoliert und mit dem Restriktionsenzym EcoRI gespalten, um das Plasmid durch anschließende Agarosegel-Elektrophorese zu überprüfen. Das erhaltene Plasmid wurde pT-pfkAexp genannt. Es ist in Figur 1 dargestellt.

### Beispiel 4

### Herstellung des Stammes ATCC13032ΔilvA/pND-D2, pT-pfkAexp

Nach Elektroporation (Tauch et.al., 1994, FEMS Microbiological Letters, 123:343-347) des Plasmids pND-D2 in den C. glutamicum Stamm ATCC13032ΔilvA und anschließender Selektion auf LB-Agar (Lennox, 1955, Virology, 1:190-206), der mit 25 µg/ml Kanamycin supplementiert worden war, wurde der Stamm ATCC13032ΔilvA/pND-D2 erhalten.

Nach Elektroporation des Plasmids pT-pfkAexp (Beispiel 3) in den C. glutamicum Stamm ATCC13032ΔilvA/pND-D2 und anschließender Selektion auf LB-Agar, der mit 25 µg/ml Kanamycin und 10 µg/ml Tetracyclin supplementiert worden war, wurde der Stamm ATCC13032ΔilvA/pND-D2, pT-pfkAexp erhalten.

### Beispiel 5

### Herstellung von Pantothensäure

Die Bildung von Pantothenat durch die C. glutamicum Stämme ATCC13032ΔilvA/pND-D2 und ATCC13032ΔilvA/pND-D2, pT-pfkAexp wurde in Medium CGXII (Keilhauer et al., 1993, Journal of Bacteriology, 175:5595-5603; Tabelle 1) geprüft, das mit 25 µg/ml Kanamycin, 2 mM Isoleucin und im Falle des Stammes ATCC13032ΔilvA/pND-D2, pT-pfkAexp mit zusätzlich 10 µg/ml Tetracyclin supplementiert worden war.

Dieses Medium wird im Folgenden als C. glutamicum-Testmedium bezeichnet. Je 50 ml frisch angesetztes C. glutamicum-Testmedium wurden aus einer 16 Stunden alten Vorkultur des gleichen Mediums dergestalt angeimpft, dass die optische Dichte der Kultursuspension (o.D.₅₈₀) bei Inkubationsbeginn 0,1 betrug. Die Kulturen wurden bei 30°C und 130 U/min bebrütet. Nach 5 stündiger Inkubation wurde IPTG (Isopropyl β-D-thiogalactosid) in einer Endkonzentration von 1 mM hinzugefügt. Nach 48stündiger Inkubation wurde die optische Dichte (o.D.₅₈₀) der Kultur bestimmt und anschließend die Zellen durch 10minütige Zentrifugation bei 5000 g entfernt und der Überstand sterilfiltriert.

Zur Bestimmung der optischen Dichte wurde ein Novaspec II Photometer der Firma Pharmacia (Freiburg, Deutschland) bei einer Messwellenlänge von 580 nm eingesetzt.

Die Quantifizierung des D-Pantothenats im Kulturüberstand erfolgte mittels Lactobacillus plantarum ATCC 8014 nach Angaben des Handbuchs der Firma DIFCO (DIFCO MANUAL, 10^{th} Edition, S. 1100-1102; Michigan, USA). Für die Kalibrierung wurde das Hemicalciumsalz von Pantothenat der Firma Sigma (Deisenhofen, Deutschland) verwendet.

Das Ergebnis ist in Tabelle 2 dargestellt.

**Tabelle 1**

| Substanz | Menge pro Liter | Bemerkung |
|---|---|---|
| (NH₄)₂ SO₂ | 20 g | |
| Harnstoff | 5 g | |
| KH₂PO₄ | 1 g | |
| K₂HPO₄ | 1 g | |
| MgSO₄ * 7 H₂O | 0.25 g | |
| MOPS | 42 g | |
| CaCl₂ | 10 mg | |
| FeSO₄ * 7 H₂O | 10 mg | |
| MnSO₄ * H₂O | 10 mg | |
| ZnSO₄ * 7 H₂O | 1 mg | |
| CuSO₄ | 0.2 mg | |
| NiCl₂ * 6 H₂O | 0.02 mg | |
| Biotin | 0.5 mg | |
| Glukose | 40 g | separat autoklavieren |
| Protocatechusäure | 0.03 mg | sterilfiltrieren |

**Tabelle 2**

| Stamm | Zelldichte oD₅₈₀ | Konzentration (ng/ml) |
|---|---|---|
| ATCC13032ΔilvA/pND-D2 | 11,5 | 47,9 |
| ATCC13032ΔilvA/pND-D2, pT-pfkAexp | 12,8 | 119,9 |

### Folgende Abbildungen sind beigefügt:

- Figur 1:: Karte des Plasmids pT-pfkAexp
- Figur 2:: Karte des Plasmids pEC-T18mob2

Die verwendeten Abkürzungen und Bezeichnungen haben folgende Bedeutung.
- Tet:: Resistenzgen für Tetracyclin
- oriV:: Plasmidkodierter Replikationsursprung von E. coli
- RP4mob:: mob Region zur Mobilisierung des Plasmides
- rep:: Plasmidkodierter Replikationsursprung aus C. glutamicum Plasmid pGA1
- per:: Gen zur Kontrolle der Kopienzahl aus pGA1
- lacZ-alpha:: lacZα-Genfragment (N-Terminus) des β-Galactosidase Gens
- 'lacZa':: 3'Ende des lacZα-Genfragments
- lacZ-alpha':: 5'Ende des lacZα-Genfragments
- pfkA:: pfkA-Gen aus C. glutamicum ATCC13032

- BamHI:: Schnittstelle des Restriktionsenzyms BamHI
- EcoRI:: Schnittstelle des Restriktionsenzyms EcoRI
- HindIII:: Schnittstelle des Restriktionsenzyms HindIII
- KpnI:: Schnittstelle des Restriktionsenzyms KpnI
- PstI:: Schnittstelle des Restriktionsenzyms PstI
- PvuI:: Schnittstelle des Restriktionsenzyms PvuI
- SalI:: Schnittstelle des Restriktionsenzyms SalI
- SacI:: Schnittstelle des Restriktionsenzyms SacI
- SmaI:: Schnittstelle des Restriktionsenzyms SmaI
- SphI:: Schnittstelle des Restriktionsenzyms SphI
- XbaI:: Schnittstelle des Restriktionsenzyms XbaI
- XhoI:: Schnittstelle des Restriktionsenzyms XhoI

## Patentansprüche

1. Verfahren zur Herstellung von D-Pantothensäure durch Fermentation coryneformer Bakterien, **dadurch gekennzeichnet, dass** man Bakterien einsetzt, in denen man die für die Phosphofructokinase (EC 2.7.1.11) kodierende Nucleotidsequenz (pfkAn) verstärkt, insbesondere überexprimiert.

2. Verfahren gemäß Anspruch 1, **dadurch gekennzeichnet, dass** man Bakterien einsetzt, in denen man zusätzlich weitere Gene des Biosyntheseweges der D-Pantothensäure verstärkt.

3. Verfahren gemäß Anspruch 1, **dadurch gekennzeichnet, dass** man Bakterien einsetzt, in denen die Stoffwechselwege zumindest teilweise ausgeschaltet sind, die die Bildung der D-Pantothensäure verringern.

4. Verfahren gemäß Anspruch 1, **dadurch gekennzeichnet, dass** man einen mit einem Plasmidvektor transformierten Stamm einsetzt, und der Plasmidvektor die für die Phosphofructokinase kodierende Nucleotidsequenz trägt.

5. Verfahren gemäß Anspruch 4, **dadurch gekennzeichnet, dass** man mit dem Plasmid pT-pfkAexp, hinterlegt unter der Nummer DSM 13253 bei der DSMZ, transformierte Bakterien einsetzt.

6. Verfahren gemäß Anspruch 1, **dadurch gekennzeichnet, dass** man gleichzeitig das für die Ketopantoat-Hydroxymethyltransferase kodierende panB-Gen verstärkt.

7. Verfahren gemäß Anspruch 1, **dadurch gekennzeichnet, dass** man gleichzeitig das für die Pantothenat-Synthetase kodierende panC-Gen verstärkt.

8. Verfahren gemäß Anspruch 1, **dadurch gekennzeichnet, dass** man gleichzeitig das für die Dihydroxysäure-Dehydratase kodierende ilvD-Gen verstärkt.

9. Verfahren gemäß einem oder mehreren der vorhergehenden Ansprüche, **dadurch gekennzeichnet, dass** man die genannten Gene in coryneformen Bakterien verstärkt, die bereits D-Pantothensäure produzieren.

10. Verfahren zur fermentativen Herstellung von D-Pantothensäure gemäß einem oder mehreren der vorhergehenden Ansprüche, **dadurch gekennzeichnet, dass** man folgende Schritte durchführt:
a) Fermentation der D-Pantothensäure produzierenden Bakterien, in denen zumindest das für die Phosphofructokinase kodierende Gen verstärkt wird,
b) Anreicherung der D-Pantothensäure im Medium oder in den Zellen der Bakterien und
c) Isolieren der produzierten D-Pantothensäure.

11. Coryneforme Bakterien, in denen man die für die Phosphofructokinase (EC 2.7.1.11) kodierenden Nucleotidsequenzen (pfkA-Gen) verstärkt, insbesondere überexprimiert.

12. Corynebacterium glutamicum DSM5715/pT-pfkAexp hinterlegt unter der Nummer DSM 13253 bei der DSMZ, Braunschweig.
